# EUROPEAN PATENT APPLICATION

(11) **EP 1 964 566 A1**
(43) Date of publication of application: **03.09.2008**
(21) Application number: 06835070.1
(22) Date of filing: 21.12.2006
(51) Int. Cl.: A61K 31/519, A61K 9/16, A61K 9/20, A61K 9/48, A61K 47/12, A61P 7/00, A61P 13/02

(54) **PREPARATION WITH IMPROVED BIOABSORBABILITY OF SAPROPTERIN HYDROCHLORIDE**

(30) Priority: 22.12.2005 JP 2005369136
(71) Applicant: Asubio Pharma Co., Ltd., Minato-ku Tokyo 107-8541 (JP)
(72) Inventor: OKITSU, Mitsuhito, Mishima-gun Osaka 618-8513 (JP); OGAT, Atsuto, Mishima-gun Osaka 618-8513 (JP); TANI, Yoshihiro, Mishima-gun, Osaka 618-8513 (JP)
(74) Representative: Albrecht, Thomas
(86) International application number: PCT/JP2006/325504
(87) International publication number: WO 2007/072911

(57) **Abstract**

A method of significant improvement for the bioavailability of orally administered sapropterin hydrochloride, a therapeutic agent used to treat BH4-responsive hyperphenylalaninemia, is provided. Also provided is a preparation for oral administration or ingestion having an improved bioavailability of sapropterin hydrochloride. Specifically, the preparation having an improved bioavailability of sapropterin hydrochloride contains sapropterin hydrochloride and an absorption promoter including an organic carboxylic acid having more than one carboxyl group in its molecule. The organic carboxylic acid having more than one carboxyl group in its molecule is selected from tartaric acid, citric acid, malic acid and optically active substances thereof, racemic mixtures thereof, anhydrides thereof, hydrates thereof or mixtures thereof. The amount by weight of the organic carboxylic acid is 0.1 to 100 times that of sapropterin hydrochloride.

## Description

### TECHNICAL FIELD

The present invention relates to a preparation that achieves increased bioavailability, specifically, increased absorption from the gastrointestinal tract, of sapropterin hydrochloride, a therapeutic agent used in the treatment of atypical hyperphenylalaninemia and tetrahydrobiopterin-responsive phenylketonuria.

### BACKGROUND ART

The following formula (I):

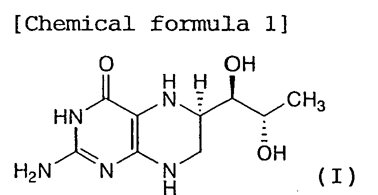

represents (R)-tetrahydrobiopterin (general name, sapropterin; chemical name, (R)-2-amino-6-[(1R,2S)-1,2-dihydropropyl]-5,6,7,8-tetrahydro-4(3H)-pteridinone; referred to as BH4, hereinafter), the natural form of tetrahydrobiopterin. (R)-tetrahydrobiopterin is the biological compound that acts as a cofactor essential for biological hydroxylation reactions and oxygenase reactions. Its dihydrochloride (sapropterin hydrochloride) was first developed in Japan as a treatment for atypical hyperphenylalaninemia caused by BH4 deficiency and approved in 1992. The compound is currently marketed under a trade name of Biopten^{®} (registered trademark).

One characteristic property of sapropterin is its extremely low oral bioavailability (about 1 to 2%). Atypical hyperphenylalaninemia caused by BH4 deficiency that the drug is intended to treat is a congenital metabolic disorder that has been considered an orphan disease, affecting a very small number of patients. Since the disease causes serious symptoms and no effective cure has been found for it, sapropterin is the only pharmaceutical product used to counteract the disease despite its low oral absorption.

When sapropterin hydrochloride is used to treat atypical hyperphenylalaninemia caused by BH4 deficiency (dihydrobiopterin synthase deficiency and dihydropteridine reductase deficiency), the drug is typically administered at a daily dose of 2 to 5 mg/kg. However, the dose can vary from a range of 6 to 15 mg/day for a 3kg newborn baby to a range of 120 to 300 mg/day for a 60kg adult. When it is desired to administer the drug in a single formulation, the dose can vary depending on the symptoms of the patients. Hence, the dose range of sapropterin hydrochloride is significantly wider than that of other drugs.

In western countries, clinical studies have recently been conducted to use sapropterin in the treatment of BH4-responsive phenylketonuria associated with hyperphenylalaninemia. According to these studies, sapropterin needs to be administered at a high daily dose of 10 mg/kg (Non-Patent Document 1). Sapropterin is also known to act as a cofactor of biological nitric oxide (NO) synthase and is reported to improve endothelium-dependent vasodilation in patients with hyperlipidemia (Non-Patent Documents 2 and 3), hypertension (Non-Patent Document 4), cardiac failure (Non-Patent Document 5) and chronic renal failure (Non-Patent Document 6). Similar effects are reported in chronic smokers (Non-Patent Document 7). The compound is also reported to improve erectile dysfunction (Non-Patent Document 8). Thus, sapropterin has a possibility of being developed not only as pharmaceutical products to treat various cardiovascular diseases and metabolic syndromes, but also as functional supplements that can prevent the onset and progress of these diseases.
Nonetheless, the cost of sapropterin produced by the currently available techniques is far from economical (Patent Document 1, Non-Patent Document 9). Thus, to develop sapropterin-based pharmaceutical products that have new therapeutic uses, it is extremely important to decrease the production cost of the compound as well as to increase its bioavailability.

In general, orally administered sapropterin hydrochloride is absorbed into the body from the gastrointestinal tract, especially from the intestinal tract, but only in small amounts. The low absorption of the drug causes its bioavailability low.
Sapropterin hydrochloride is highly susceptible to oxidation and is therefore generally formulated with an antioxidant, such as ascorbic acid and L-cysteine. Even though the stability of sapropterin hydrochloride is ensured by the addition of the antioxidants, the bioavailability of the compound remains extremely low (1 to 2%) due to its low absorption from the intestinal tract.

From a physiologic anatomical point of view, a drug administered to the body is absorbed through two different pathways: the transcellular pathway in which the drug is transported across the cytoplasm, and the paracellular pathway in which the drug is transported through the intercellular junction known as the tight junction (TJ) to the lateral intercellular space. TJs serve as the barrier to the passage of drugs, so that the drug permeability through the paracellular pathway is generally kept extremely low, leaving the transcellular pathway through the cytoplasm as the major permeation route.

The above discussion has naturally led to the pH partition theory that predicts that nonionic, lipophilic drugs are more readily absorbed from the intestinal tract. However, some evidences suggest that certain drugs are absorbed from the intestinal tract in their ionized forms: not all cases of the intestinal drug absorption can be explained by the ion-based theory alone (Non-Patent Document 10).

Accordingly, if the permeability barrier function of the TJs can be decreased or if the TJs can be opened, ionic drugs will be more readily transported through the paracellular pathway by passive transportation and, as a result, the intestinal permeability of ionic drugs can be increased.

Although sapropterin hydrochloride is a highly water-soluble compound having a low molecular weight of 314, the compound is classified as an ionic compound because it is hardly absorbed from the intestinal tract if administered in a typical oral preparation. Thus, the intestinal absorption and, thus, the bioavailability of this orally administered compound can be increased if a technique can be developed to open the TJs.

Some studies propose the use of chelating agents, such as EDTA, that trap Ca²⁺ to open the TJs because Ca²⁺ is believed to be involved in the formation of TJs. Many still support that this theory works. As the structures of intercellular adhesion molecules are made clear recently; however, it has been suggested that the opening of TJs is not simply caused by the chelating action (Non-Patent Documents 11 and 12).

The adhesion junctions and desmosomes in the intercellular spaces are filled with glycoprotein-rich adhesion molecules and are reinforced by flexible microfilament cytoskeletons. It is believed that one of the primary functions of actin and myosin, the components of the microfilaments, is to pull the cell membrane inward during cell divisions. It has also been shown that the two proteins are tightly connected by membrane-lining protein.

The intestinal epithelium also has highly developed cytoskeletons. According to one proposed mechanism of TJ opening, intracellular Ca²⁺, a second messenger that controls the actomyosin system, forms a complex with calmodulin. The Ca²⁺/calmodulin complex activates myosin light chain kinase (MLCK). The activation of MLCK and the activation of protein kinase C (PKC) involved in protein phosphorylation cause the contraction of the cytoskeleton, which in turn causes the TJs to open.

Hayashi et al. recently reported that organic carboxylic acids, such as tartaric acid and citric acid, also have an activity to open the paracellular pathway (Non-Patent Document 13). They propose the underlying mechanism as follows: the organic carboxylic acids decrease the ATP levels and acidity of the cytoplasm. The cytoplasmic acidosis and the resulting decrease in the ATP levels cause an elevation in the intracellular Ca²⁺ concentration. The elevated intracellular Ca²⁺ concentration induces cell contraction, thus causing the paracellular pathway (TJs) to open.

Patent Document 1: US Patent No. 4,713,453
Non-Patent Document 1: Molecular Genetics and Metabolism, vol. 77, 304 (2002)
Non-Patent Document 2: J. Clin. Invest., vol. 99, 41 (1997)
Non-Patent Document 3: Heart, vol. 87, 264 (2002)
Non-Patent Document 4: American Journal of Hypertension, vol. 15, 326 (2002)
Non-Patent Document 5: J. Cardio. Pharmacol., vol. 39, 363 (2002) Non-Patent Document 6: Nephrol Dial Transplant, vol. 17, 1032 (2002) Non-Patent Document 7: J. A. C. C., vol. 35, 71 (2000)
Non-Patent Document 8: Asian J. Androl., vol. 2, 159 (2006)
Non-Patent Document 9: Hervetica. Chimica. Acta., vol. 68, 1639 (1985)
Non-Patent Document 10: Scinence of Evaluating and Improving Drug Bioavailability (edited by Yuichiro Sugiyama, Gendai Iryo Sha)
Non-Patent Document 11: Am. J. Physiol., 253, C171-C175 (1987)
Non-Patent Document 12: Am. J. Physiol., 253, C854-C861 (1987)
Non-Patent Document 13: J. Controlled Release, vol. 62, 141 (1999)

In consideration of the above-described aspects of intestinal drug absorption, the present inventors contemplated that the paracellular pathway (TJs) could be opened and, thus, the intestinal absorption of orally administered sapropterin hydrochloride could be facilitated by formulating the compound with organic carboxylic acids. In particular, we orally administered to subjects sapropterin hydrochloride formulated with organic carboxylic acids having more than one carboxyl group, such as tartaric acid, citric acid and malic acid, and examined the absorption of sapropterin hydrochloride into the body.

Consequently, we demonstrated that the intestinal absorption and, thus, the bioavailability of sapropterin hydrochloride were significantly improved. This finding ultimately led to the present invention.

### DISCLOSURE OF THE INVENTION

### PROBLEMS TO BE SOLVED BY THE INVENTION

In view of the above-described present state of the art, it is an object of the present invention to provide a method for significantly improving the bioavailability of orally administered or ingested sapropterin hydrochloride.
It is another object of the present invention to provide an oral preparation for use as a functional supplement or a pharmaceutical product having an increased bioavailability of sapropterin hydrochloride.

### MEANS FOR SOLVING THE PROBLEMS

To achieve the above-described objects, one aspect of the present invention concerns the following:
(1) a method for improving a bioavailability of sapropterin hydrochloride, the method including adding to sapropterin hydrochloride an organic carboxylic acid having more than one carboxyl group in its molecule for oral administration or ingestion.

Specifically, the invention comprises the following:
(2) the method for improving a bioavailability of sapropterin hydrochloride according to (1) above, wherein the organic carboxylic acid having more than one carboxyl group in its molecule is a dicarboxylic acid or a tricarboxylic acid;
(3) the method for improving a bioavailability of sapropterin hydrochloride according to (1) above, wherein the organic carboxylic acid having more than one carboxyl group in its molecule is added either alone or as a mixture of two or more carboxylic acids;
(4) the method for improving a bioavailability of sapropterin hydrochloride according to (1), (2) or (3) above, wherein an amount (by weight) of the organic carboxylic acid having more than one carboxyl group in its molecule is 0.1 to 100 times that of sapropterin hydrochloride;
(5) the method for improving a bioavailability of sapropterin hydrochloride according to (1), (2) or (3) above, wherein the organic carboxylic acid having more than one carboxyl group in its molecule is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof; and
(6) the method for improving a bioavailability of sapropterin hydrochloride according to (1), (2), (3) or (4) above, wherein the organic carboxylic acid having more than one carboxyl group in its molecule is selected from tartaric acid, citric acid, malic acid, and optically active substances thereof, racemic mixtures thereof, anhydrides thereof, hydrates thereof or mixtures thereof.

Another aspect of the present invention concerns the following:
(7) an absorption promoter for improving a bioavailability of orally administered or ingested sapropterin hydrochloride, the absorption promoter including an organic carboxylic acid having more than one carboxyl group in its molecule.
   Specifically, the invention comprises the following:
(8) the absorption promoter according to (7) above, wherein the organic carboxylic acid having more than one carboxyl group in its molecule is a dicarboxylic acid or a tricarboxylic acid;
(9) the absorption promoter according to (7) or (8) above, wherein the organic carboxylic acid having more than one carboxyl group in its molecule is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof; and
(10) the absorption promoter according to (7) or (8) above, wherein the organic carboxylic acid having more than one carboxyl group in its molecule is selected from tartaric acid, citric acid, malic acid, and optically active substances thereof, racemic mixtures thereof, anhydrides thereof, hydrates thereof or mixtures thereof.

Still another aspect of the present invention concerns the following:
(11) a preparation having an improved bioavailability of sapropterin hydrochloride, containing sapropterin hydrochloride and an absorption promoter including an organic carboxylic acid having more than one carboxyl group in its molecule.
   Specifically, the invention comprises the following:
(12) the preparation having an improved bioavailability of sapropterin hydrochloride according to (11) above, wherein an amount (by weight) of the organic carboxylic acid having more than one carboxyl group in its molecule is 0.1 to 100 times that of sapropterin hydrochloride;
(13) the preparation having an improved bioavailability of sapropterin hydrochloride according to (11) or (12) above, wherein the organic carboxylic acid having more than one carboxyl group in its molecule is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof;
(14) the preparation having an improved bioavailability of sapropterin hydrochloride according to (11) or (12) above, wherein the organic carboxylic acid having more than one carboxyl group in its molecule is selected from tartaric acid, citric acid, malic acid, and optically active substances thereof, racemic mixtures thereof, anhydrides thereof, hydrates thereof or mixtures thereof; and
(15) the preparation according to any of (11) to (14) above, prepared in the form of tablets, capsules, granules, fine grains or soft capsules.

The present invention improves the intestinal absorption and, thus, the bioavailability of sapropterin hydrochloride, which could otherwise achieve a low bioavailability of at most 1 to 2% even if orally administered, by adding to it an absorption promoter comprising an organic carboxylic acid having more than one carboxyl group in its molecule. One feature of the present invention is that sapropterin hydrochloride is formulated with an organic carboxylic acid having more than one carboxyl group in its molecule.

Another feature of the present invention is that the organic carboxylic acid that has more than one carboxyl group in its molecule and is used as the absorption promoter of sapropterin hydrochloride is a highly safe organic carboxylic acid that has already been approved as an additive to pharmaceutical products or food products.

### EFFECT OF THE INVENTION

The present invention makes it possible to significantly improve the intestinal absorption and, thus, the bioavailability of sapropterin hydrochloride, a compound that could otherwise achieve a low bioavailability of at most 1 to 2% when orally administered or ingested.
As a result, the oral dose of sapropterin hydrochloride required to achieve the effective blood concentration of the compound can be reduced almost by half. The reduction in the dose of sapropterin hydrochloride to patients with atypical hyperphenylalaninemia ensures safety during the long-term drug administration and is therefore highly advantageous.
Since the amount of sapropterin hydrochloride used in the preparation can also be decreased, the pharmaceutical preparation can be produced at a significantly reduced cost. This is advantageous given the high production cost of active ingredients. The reduced production cost also facilitates development of sapropterin hydrochloride as pharmaceutical products that find new therapeutic uses, such as the treatment of cardiovascular diseases, and as functional supplements.

### BEST MODE FOR CARRYING OUT THE INVENTION

Sapropterin hydrochloride (dihydrochloride), the active ingredient administered or ingested according to the present invention and represented by the formula (1), was developed as an oral treatment for atypical hyperphenylalaninemia caused by BH4 deficiency. The compound was approved in 1992 and is currently marketed under a trade name of Biopten (registered trademark).
As described above, sapropterin hydrochloride is hardly absorbed from the intestinal tract when orally administered: it has a low bioavailability of at most 1 to 2%.
The present invention addresses the problem of the low intestinal absorption of the compound by the addition of an organic carboxylic acid having more than one carboxyl group in its molecule.

The organic carboxylic acid having more than one carboxyl group in its molecule that is to be added to sapropterin hydrochloride in the present invention may be a dicarboxylic acid or a tricarboxylic acid. Specific examples include tartaric acid, citric acid, malonic acid, maleic acid, fumaric acid, adipic acid, malic acid and mixtures thereof. Their optically active substances, racemic mixtures, anhydrides and hydrates are also included.
Of these organic carboxylic acids, tartaric acid, citric acid and malic acid are particularly preferred, as are their optically active substances, racemic mixtures, anhydrides and hydrates.

The preferred acids, tartaric acid, citric acid and malic acid are each a highly safe compound already approved as an additive to pharmaceutical products and food products. These organic carboxylic acids are widely used in pharmaceutical compositions as stabilizers, buffers, flavoring agents, pH adjusters, isotonizing agents, foaming agents, disintegrating agents, excipients, dispersers, solubilizers, antioxidants, preservatives and other purposes. However, their ability to improve the intestinal absorption of orally administered or ingested sapropterin hydrochloride has not been known until now.

The present inventors have demonstrated in our studies that these organic carboxylic acids having more than one carboxyl group in their molecules can serve as absorption promoters that increase the bioavailability of orally administered or ingested active ingredient of sapropterin hydrochloride.

The amount (by weight) of the organic carboxylic acid to serve as the absorption promoter is preferably 0.1 to 100 times that of sapropterin hydrochloride to serve as the active ingredient.
When added in an amount less than 0.1 times (by weight) the amount of sapropterin hydrochloride, the organic carboxylic acid cannot sufficiently improve the bioavailability of sapropterin hydrochloride. When the organic carboxylic acid is added in an amount more than 100 times (by weight) the amount of sapropterin hydrochloride, the bioavailability of sapropterin hydrochloride is not improved correspondingly and the excess organic carboxylic acid is wasted.

Traditionally, the effective daily dose of sapropterin hydrochloride for the treatment of atypical hyperphenylalaninemia caused by BH4 deficiency has been in the range of 2 to 5 mg/kg. According to the present invention, the oral dose of sapropterin hydrochloride required to maintain the effective blood concentration of the compound, as well as the amount of the organic carboxylic acid added as the absorption promoter, is determined by considering the significantly improved bioavailability of the compound.

To find new therapeutic uses of sapropterin hydrochloride, such as in the treatment of cardiovascular diseases, the oral dose of sapropterin hydrochloride and the amount of the organic carboxylic acid added as the absorption promoter are determined by considering the effective blood concentration of the compound to treat the target disease.

The preparation of the present invention containing sapropterin hydrochloride along with the organic carboxylic acid to serve as the absorption promoter may be provided in the form of tablets, capsules, granules, fine grains, soft capsules or dry syrups.
Of these forms, powdered preparations, such as granules and fine grains, and dry syrups are particularly preferred since these preparations can be used to administer sapropterin hydrochloride to patients of varying ages, from newborn babies to adults, in one preparation.
Specifically, tablets and capsules are unsuitable for use in patients such as babies, who cannot swallow, or aged people, who have difficulty swallowing. On the other hand, powdered preparations, such as granules and fine grains, and dry syrups can be orally taken or ingested directly, or they may be prepared as a solution upon use.
Tablets and capsules can be used in patients with high body weights such as adults and in new therapeutic uses.

These preparations can be prepared by any of the techniques commonly used in the field of pharmaceutical preparations and may further contain other pharmaceutically acceptable additives as described in Japanese Pharmacopoeia, including stabilizers, buffers, flavoring agents, pH adjusters, isotonizing agents, foaming agents, disintegrating agents, excipients, dispersers, solubilizers, antioxidants, preservatives and coloring agents.

### EXAMPLES

The present invention will now be described in detail with reference to Test Examples and Examples.

### Test Example 1:

### Examination of blood concentration in oral administration of sapropterin hydrochloride with organic carboxylic acid having more than one carboxyl group (Part 1)

The ability of organic carboxylic acids having more than one carboxyl group to increase the blood concentration of sapropterin was examined in a rat model by adding L-tartaric acid or citric acid to sapropterin hydrochloride.

### [Method]

Male SD rats (7 weeks old, weighing 229 to 267 g, 5 animals in each group) were orally administered 30 mg/kg of sapropterin hydrochloride along with L-tartaric acid or citric acid. L-tartaric acid was administered at doses of 375 mg/kg (0.5 mol solution at 5 mL/kg, 12.5 times the weight of sapropterin hydrochloride) and 75 mg/kg (0.1 mol solution at 5 ml/kg, 2.5 times the weight of sapropterin hydrochloride).
Citric acid was administered at doses of 480 mg/kg (0.5 mol solution at 5 mL/kg, 16.0 times the weight of sapropterin hydrochloride) and 96 mg/kg (0.1 mol solution at 5 mL/kg, 3.2 times the weight of sapropterin hydrochloride).
As a control group, rats were administered sapropterin hydrochloride formulated with physiological saline, without organic carboxylic acids.
Blood samples were taken 0.5, 1, 2 and 4 hours after the administration and analyzed for the concentration of sapropterin free base.

### [Analysis of blood concentration]

Using a high-performance liquid chromatography system equipped with a fluorescence detector, the blood concentration of sapropterin was analyzed according to the method described by Tani et al (J. Chromatography, 617, 249-255 (1993)).
The high-performance liquid chromatography system is composed of two pumps (LC-6A, Shimadzu), a system controller (SCL-6B, manufactured by Shimadzu Corporation), an autoinjector (SIL-6B, manufactured by Shimadzu Corporation), a reverse-phase column (Cosmosil 5C18, 250 x 4.6 mm, manufactured by Nacalai Tesque, Inc.), two column ovens (CTO-6A, manufactured by Shimadzu Corporation), a reaction coil and a fluorescence detector (RF-550, manufactured by Shimadzu Corporation).

The excitation wavelength and the fluorescence wavelength of the fluorescence detector were fixed at 350 nm and 440 nm, respectively. The temperatures of the analysis column and the reaction coil were maintained at 40°C and 80°C, respectively. For the measurement of sapropterin concentration, a standard peak of a known standard substance was first drawn by using an integrator (C-R4AX, manufactured by Shimadzu Corporation). The area and the concentration obtained from the chart were used to create a file for analysis (External standard method).

The mobile phase used was 0.1 mM sodium phosphate buffer (pH 3.0) containing 5% (v/v) methanol, 3 mM sodium octylsulfate, 0.1 mM disodium EDTA and 0.1 mM ascorbic acid. The flow rate was 1.0 mL/min.
Of different pterins separated by the reverse-phase column in the method, non-fluorescent reduced pterins (such as BH4) were converted into fluorescent oxidized pterins in the reaction coil with NaNO₂ (5 mM, flow rate = 1.0 mL/min) prior to the detection by the fluorescence detector.

### [Results]

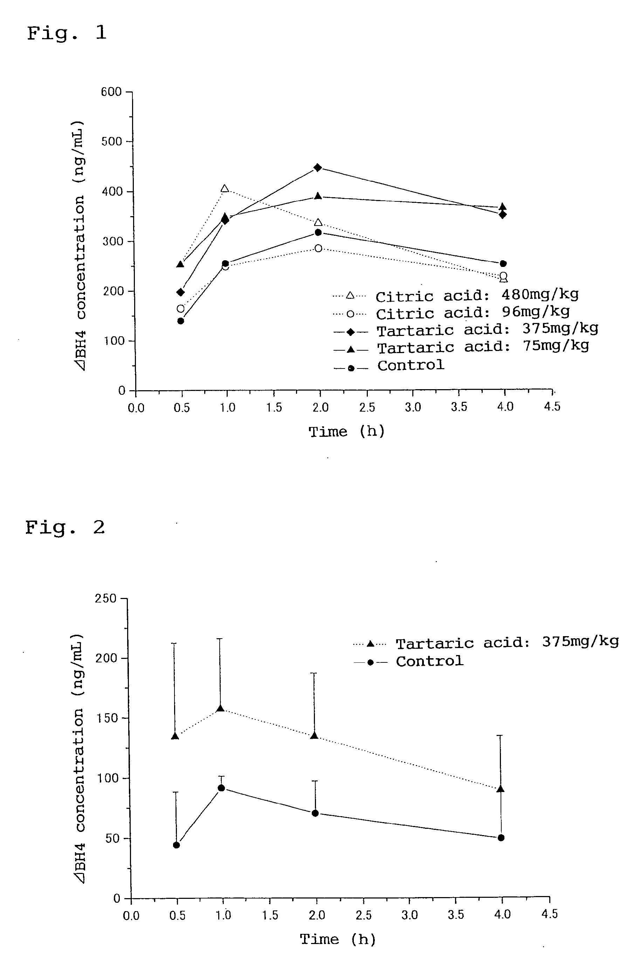

The results are shown in Fig. 1.
As can be seen from the results of Fig. 1, the blood concentration of sapropterin was higher in the groups administered sapropterin hydrochloride with L-tartaric acid or citric acid than in the control group administered sapropterin hydrochloride alone. The increase was significant in the groups receiving 375 ml/kg or 75 ml/kg of L-tartaric acid or 480 mg/kg of citric acid. In these groups, the blood concentration of propterin remained high even 4 hours after the administration.

### Test Example 2:

### Examination of blood concentration in oral administration of sapropterin hydrochloride with organic carboxylic acid having more than one carboxyl group (Part 2)

The results of Test Example 1 indicate that the increase in the absorption of orally administered sapropterin hydrochloride was most significant in the groups receiving L-tartaric acid.
As in Test Example 1, sapropterin hydrochloride was administered to male SD rats (7 weeks old, weighing 229 to 267 g, 8 animals in each group) at a dose of 10 mg/kg (the clinical dose used in the treatment of BH4-responsive phenylketonuria), along with 125 mg/kg (12.5 times as much (by weight)) of L-tartaric acid. The improvement in the bioavailability of orally administered sapropterin was examined.
As in Test Example 1, a control group was administered sapropterin hydrochloride with physiological saline, without organic carboxylic acid.

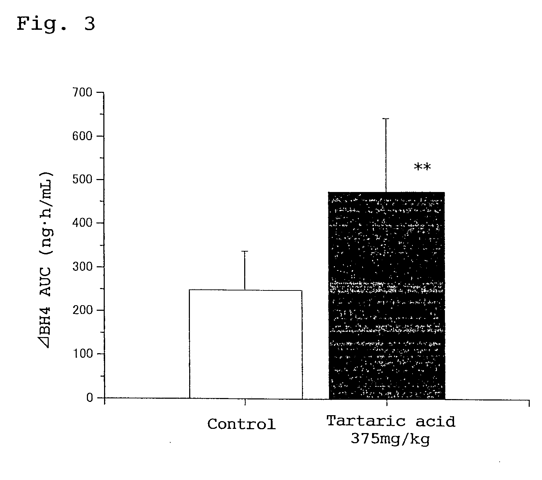

The results are shown in Figs. 2 and 3.
Fig. 2 shows the change in the blood concentration of sapropterin 0.5, 1, 2 and 4 hours after the administration for each group. As shown, the blood concentration of sapropterin remained higher in the receiving group of L-tartaric acid than in the control group.
Fig. 3 shows the area under concentration (AUC) for each group. As shown, the AUC of the L-tartaric acid group is about twice that of the control group, indicating a significant improvement in the bioavailability of sapropterin in the group receiving L-tartaric acid.

Specific examples of the preparation of the present invention will now be described with reference to the above-described Test Examples. The present invention is not limited to these preparations, however.

### Preparation Example 1:

Granule: Using a known technique, a granule preparation having the following composition was prepared:

| | |
|---|---|
| sapropterin hydrochloride | 1 part |
| D-mannitol | 8 parts |
| povidone | 0.1 parts |
| low-substituted hydroxypropylcellulose | 0.5 parts |
| ascorbic acid | 0.05 parts |
| L-cysteine hydrochloride | 0.02 parts |
| L-tartaric anhydride | 0.5 - 2.5 parts |
| riboflavin (coloring agent) | a small amount |

### Preparation Example 2:

Tablet: Using a known technique, a tablet preparation having the following composition was prepared:

| | |
|---|---|
| sapropterin hydrochloride | 1 part |
| lactose | 80 parts |
| corn starch | 20 parts |
| carboxypropylcellulose | 3 parts |
| ascorbic acid | 0.05 parts |
| L-cysteine hydrochloride | 0.02 parts |
| magnesium stearate | 1 part |
| L-tartaric anhydride | 0.5 - 2.5 parts |
| riboflavin (coloring agent) | a small amount |

### Preparation Example 3:

Capsule: Using a known technique, a capsule preparation having the following composition was prepared:

| | |
|---|---|
| sapropterin hydrochloride | 1 part |
| avicel | 5 parts |
| ascorbic acid | 0.05 parts |
| L-cysteine hydrochloride | 0.02 parts |
| magnesium stearate | 0.05 parts |
| L-tartaric anhydride | 0.5 - 2.5 parts |
| riboflavin (coloring agent) | a small amount |

### INDUSTRIAL APPLICABILITY

As described above, the present invention provides a safe oral sapropterin hydrochloride preparation that contains an organic carboxylic acid having more than one carboxyl group and thus has a significantly improved bioavailability of sapropterin. Such an organic carboxylic acid is approved as an additive to pharmaceutical products.
By using the preparation of the present invention, the extremely low bioavailability of orally administered sapropterin hydrochloride (1 to 2%) can be significantly improved and the oral dosage of sapropterin hydrochloride required to maintain the effective blood concentration can be decreased almost by half.

The fact that the dosage of sapropterin hydrochloride required to maintain the effective blood concentration can be decreased almost by half is highly advantageous in ensuring safety in patients with atypical hyperphenylalaninemia receiving long-term administration of the compound.
In addition, the pharmaceutical sapropterin preparation can be produced at a significantly reduced cost, which is advantageous given the high production cost of sapropterin. The reduced production cost also facilitates development of sapropterin hydrochloride as pharmaceutical products that find new therapeutic uses, such as the treatment of cardiovascular diseases, and as functional supplements. Therefore, the present invention should make a significant contribution to medical treatment and health care.

### BRIEF DESCRIPTION OF THE DRAWINGS

Fig. 1 is a diagram showing the change in the blood concentration of sapropterin in Test Example 1.
Fig. 2 is a diagram showing the change in the blood concentration of sapropterin in Test Example 2.
Fig. 3 is a diagram showing the area under concentration (AUC) in Test Example 2.

## Claims

1. A method for improving a bioavailability of sapropterin hydrochloride, the method comprising adding to sapropterin hydrochloride an organic carboxylic acid having more than one carboxyl group in a molecule thereof for oral administration or ingestion.

2. The method for improving a bioavailability of sapropterin hydrochloride according to claim 1, wherein the organic carboxylic acid having more than one carboxyl group in a molecule thereof is a dicarboxylic acid or a tricarboxylic acid.

3. The method for improving a bioavailability of sapropterin hydrochloride according to claim 1, wherein the organic carboxylic acid having more than one carboxyl group in a molecule thereof is added either alone or as a mixture of two or more carboxylic acids.

4. The method for improving a bioavailability of sapropterin hydrochloride according to claim 1, 2 or 3, wherein an amount by weight of the organic carboxylic acid having more than one carboxyl group in a molecule is 0.1 to 100 times that of sapropterin hydrochloride.

5. The method for improving a bioavailability of sapropterin hydrochloride according to claim 1, 2, 3 or 4, wherein the organic carboxylic acid having more than one carboxyl group in a molecule thereof is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof.

6. The method for improving a bioavailability of sapropterin hydrochloride according to claim 1, 2, 3 or 4, wherein the organic carboxylic acid having more than one carboxyl group in a molecule thereof is selected from tartaric acid, citric acid, malic acid, and optically active substances thereof, racemic mixtures thereof, anhydrides thereof, hydrates thereof or mixtures thereof.

7. An absorption promoter for improving a bioavailability of orally administered or ingested sapropterin hydrochloride, the absorption promoter comprising an organic carboxylic acid having more than one carboxyl group in a molecule thereof.

8. The absorption promoter according to claim 7, wherein the organic carboxylic acid having more than one carboxyl group in a molecule thereof is a dicarboxylic acid or a tricarboxylic acid.

9. The absorption promoter according to claim 7 or 8, wherein the organic carboxylic acid having more than one carboxyl group in a molecule thereof is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof.

10. The absorption promoter according to claim 7 or 8, wherein the organic carboxylic acid having more than one carboxyl group in a molecule thereof is selected from tartaric acid, citric acid, malic acid, and optically active substances thereof, racemic mixtures thereof, anhydrides thereof, hydrates thereof or mixtures thereof.

11. A preparation having an improved bioavailability of sapropterin hydrochloride, containing sapropterin hydrochloride and an absorption promoter comprising an organic carboxylic acid having more than one carboxyl group in a molecule thereof.

12. The preparation having an improved bioavailability of sapropterin hydrochloride according to claim 11, wherein an amount by weight of the organic carboxylic acid having more than one carboxyl group in a molecule thereof is 0.1 to 100 times that of sapropterin hydrochloride.

13. The preparation having an improved bioavailability of sapropterin hydrochloride according to claim 11 or 12, wherein the organic carboxylic acid having more than one carboxyl group in a molecule thereof is selected from tartaric acid, citric acid, malonic acid, maleic acid, fumaric acid, adipic acid, malic acid, and optically active substances thereof, racemic mixtures thereof, anhydrides thereof, hydrates thereof or mixtures thereof.

14. The preparation having an improved bioavailability of sapropterin hydrochloride according to claim 11 or 12, wherein the organic carboxylic acid having more than one carboxyl group in a molecule thereof is selected from tartaric acid, citric acid, malic acid, and optically active substances thereof, racemic mixtures thereof, anhydrides thereof, hydrates thereof or mixtures thereof.

15. The preparation according to any of claims 11 to 14, prepared in the form of tablets, capsules, granules, fine grains or soft capsules.
